(19) Europäisches Patentamt
European Patent Office
Office européen des brevets

(11) **EP 4 295 148 B1**

(12) **EUROPÄISCHE PATENTSCHRIFT**

(45) Veröffentlichungstag und Bekanntmachung des Hinweises auf die Patenterteilung:
**12.11.2025  Patentblatt 2025/46**

(21) Anmeldenummer: **22702454.4**

(22) Anmeldetag: **27.01.2022**

(51) Internationale Patentklassifikation (IPC):
*G01N 33/14* (2006.01)     *C12C 11/00* (2006.01)

(52) Gemeinsame Patentklassifikation (CPC):
**G01N 33/146; C12C 11/00**

(86) Internationale Anmeldenummer:
**PCT/EP2022/051857**

(87) Internationale Veröffentlichungsnummer:
**WO 2022/175049 (25.08.2022 Gazette 2022/34)**

(54) **ÜBERWACHUNG UND STEUERUNG EINER HEFEPROPAGATION**

MONITORING AND CONTROLLING YEAST PROPAGATION

SURVEILLANCE ET RÉGULATION DE LA PROPAGATION DE LEVURES

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität:  **18.02.2021  DE 102021103928**

(43) Veröffentlichungstag der Anmeldung:
**27.12.2023  Patentblatt 2023/52**

(73) Patentinhaber: **Endress+Hauser SE+Co. KG**
**79689 Maulburg (DE)**

(72) Erfinder:
• **SCHON, Julia**
**78554 Aldingen (DE)**
• **ROSENHEIM, Julia**
**79664 Wehr (DE)**
• **BRENGARTNER, Tobias**
**79312 Emmendingen (DE)**
• **LOPATIN, Sergey**
**79540 Lörrach (DE)**

(74) Vertreter: **Endress + Hauser Group Services (Deutschland) AG+Co. KG**
**Colmarer Straße 6**
**79576 Weil am Rhein (DE)**

(56) Entgegenhaltungen:
**EP-A1- 2 846 160     US-A1- 2008 109 100**

• **ZWIETERING M H ET AL: "Modeling of the bacterial growth curve", APPLIED AND ENVIRONMENTAL MICROBIOLOGY, AMERICAN SOCIETY FOR MICROBIOLOGY, US, vol. 56, no. 6, 1 June 1990 (1990-06-01), pages 1875 - 1881, XP002664178, ISSN: 0099-2240**

## Beschreibung

Die vorliegende Erfindung betrifft ein Verfahren, insbesondere ein computerimplementiertes Verfahren, zur Steuerung und/oder Überwachung einer Hefevermehrung bzw. Hefepropagation, beispielsweise in einer großtechnischen Anlage. Insbesondere betrifft die Erfindung die Hefepropagation in einer Brauerei.

Die Herstellung von Bier umfasst mehrere komplexe Verfahrensschritte, wie das Mälzen von Getreide, das Maischen, das Läutern, das Würzekochen und das Fermentieren. Für eine zuverlässige Überwachung und/oder Steuerung sowie eine Optimierung solcher komplexen Prozesse ist die simultane Kenntnis unterschiedlichster Prozessgrößen und der jeweils zugrundeliegenden Zusammenhänge erforderlich. So ist beispielsweise aus der DE 10 2019 110 821 A1 ein Verfahren zur Bestimmung und/oder Überwachung einer Konzentration von Maltodextrin und/oder Maltose anhand der Dichte und der Schallgeschwindigkeit in einem Maischprozess bekannt geworden. Hierdurch kann die Zeitdauer zur Verzuckerung der angestellten Maische exakt bestimmt werden.

Die Würzegärung betrifft das Ansetzen oder Inokulieren von gehopfter Anstellwürze bzw. Würze mit einer Hefe. Die Hefevermehrung bzw. Hefepropagation erfolgt üblicherweise im sogenannten Batch-Betrieb, wobei in jedem Ansatz eine frische Hefekultur in der Würze angesetzt und komplett für die nachfolgende Gärung verwendet wird. Unter Hefevermehrung bzw. Propagation wird in diesem Zusammenhang die Erhöhung der Biomasse und Zellzahl der jeweiligen Hefepopulation verstanden, welche in mehreren unterschiedlichen Wachstumsphasen erfolgt. Idealerweise sollte im Ergebnis eine Hefe mit möglichst hoher Hefevitalität, d. h. hoher Gärkraft und Viabilität, d.h. Lebensfähigkeit, vorliegen. Um dies zu gewährleisten, können während des Gärprozesses verschiedene Messungen mit oder ohne Probennahme erfolgen, anhand welcher der Prozess der Hefevermehrung überwacht werden kann. Eine einheitliche oder gängige Verfahrensweise hinsichtlich der Überwachung und/oder Steuerung von Hefevermehrung ist allerdings nicht verfügbar. Eine solche ist für solch ein multivariantes, biologisches System mit einem komplexen Wachstumsverhalten auch nur schwer realisierbar.

Die EP 2 846 160 A1 beschreibt die Bierfermentation, bei der unter Anwendung von Fuzzy-Logik Modelle berechnet werden, die das Verhalten der Maische in Abhängigkeit von Messparametern wie z. B. der Temperatur beschreiben. Vorhanden ist eine Messeinheit zur Messung eines Parameters (z. B. Alkohol- oder $CO_2$-Gehalt), der mit dem Extrakt-Wert korreliert. Aus den Extrakt-Werten wird eine Extrapolation vorgenommen, um optimale und möglichst wenige Messzeitpunkte zu setzen. Die Fermentation wird dann durch Herunterkühlen beendet, wenn eine Änderung des Extrakt-Werts zu gering und ein gemessener VDK-Wert unter einem Zielwert liegt.

Die US 2008/0109100 A1 diskutiert Rechen-Modelle für die Überwachung der Fermentation.

Der Erfindung liegt die Aufgabe zugrunde, die Steuerung und/oder Überwachung einer Hefepropagation zu verbessern.

Diese Aufgabe wird gelöst durch ein Verfahren, insbesondere ein computerimplementiertes Verfahren, zur Steuerung und/oder Überwachung einer Hefevermehrung, bzw. Hefepropagation, umfassend die Verfahrensschritte des ersten Anspruchs.

Die Stammwürze in Grad Plato (°P) bezeichnet den Gehalt an gelösten Inhaltsstoffen aus Malz und Hopfen in der Würze vor Beginn der Fermentation und zu einem Startzeitpunkt der Hefevermehrung. Während der Hefevermehrung nimmt dieser Wert in Folge des Hefemetabolismus ab. Der Gehalt an gelösten Inhaltsstoffen aus Malz und Hopfen nach Beendigung der Hefevermehrung wird als Restextraktgehalt bezeichnet.

Durch Berücksichtigung eines logistischen Propagationsmodells ermöglicht es, eine Zeitdauer für den Prozess der Hefevermehrung genau vorhersagen zu können. Die Erfindung beruht auf der Erkenntnis, dass von einer Vielzahl möglicher Einflussfaktoren für die Hefevermehrung die Stammwürze und Temperatur zum Startzeitpunkt entscheidend sind. Eine Überwachung und/oder Steuerung der Hefepropagation ist somit auf einfache Art und Weise realisierbar, insbesondere kann eine Dauer für die Hefepropagation durch Vorhersage des Endzeitpunkts optimiert werden.

Eine Ausgestaltung des Verfahrens beinhaltet, dass ein freier Aminostickstoffgehalt (FAN-Gehalt) der Würze bestimmt wird, und für die Berechnung des theoretischen Endzeitpunkts anhand des Propagationsmodells berücksichtigt wird. Der FAN-Gehalt spielt insbesondere für die Stoffwechselaktivitäten der Hefe, für das Hefewachstum und den physiologischen Zustand der Hefe eine Rolle. Der FAN-Gehalt kann beispielsweise regelmäßig durch Probennahme, beispielsweise während der Durchführung von Prozesskontrollen in der Brauerei, ermittelt werden.

Eine weitere Ausgestaltung des Verfahrens beinhaltet, dass die Temperatur des Gemisches während der Hefevermehrung bestimmt bzw. gemessen wird. Die Temperatur ist ein dominierender Faktor für die Wachstumsrate der Hefezellen und beeinflusst somit die Geschwindigkeit der Hefevermehrung maßgebend. Bei Detektion einer Temperaturänderung kann beispielsweise die Vorhersage für den Endzeitpunkt der Hefevermehrung geeignet korrigiert werden.

Erfindungsgemäß wird ein Istwert für den Extraktgehalt des Gemisches während der Hefevermehrung bestimmt bzw. gemessen. Der Extraktgehalt kann beispielsweise zur Überwachung der Nährstoffversorgung der Hefe während des Prozesses der Hefevermehrung herangezogen werden.

Zudem ist es von Vorteil, wenn ein Alkoholgehalt, insbesondere ein Ethanolgehalt, des Gemisches während der Hefevermehrung bestimmt bzw. gemessen wird. Während der Hefevermehrung kommt es zu einer Anhäufung von extrazellulärem Ethanol, welches die Wachstumsgeschwindigkeit der Hefe ebenfalls beeinflusst.

In einer erfindungsgemäßen Variante wird ein Gehalt an gelöstem Sauerstoff des Gemisches während der Hefevermehrung bestimmt bzw. gemessen. Diese Größe wiederum kann zur Überprüfung und/oder Überwachung des Vorliegens einer Sättigung der Würze dienen.

Eine Ausgestaltung des Verfahrens beinhaltet, dass als Propagationsmodell ein Modell für einen Extraktgehalt in dem Gemisch verwendet wird, wobei der Extraktgehalt anhand der Stammwürze zum Startzeitpunkt, eines Zielwerts für den Restextraktgehalt und unter Berücksichtigung einer Substrataufnahmerate, einer Temperaturrate und einer Dauer für eine Lag-Phase während der Hefevermehrung ermittelt wird.

Die Vorgabe eines Zielwerts für den Restextraktgehalt dient der Gewährleistung einer ausreichenden Nährstoffversorgung und Vitalität der Hefe bis zum Endzeitpunkt der Hefepropagation.

Anhand der Substrataufnahmerate kann die spezielle Wachstumskinetik der Hefevermehrung mit in das Propagationsmodell einbezogen werden. Für die Hefevermehrung werden sämtliche Substrate der Hefe zu Beginn des Prozesses zugeführt. Damit wachsen die Hefezellen grundsätzlich bis zur Erschöpfung der Substrate.

Die Temperaturrate beeinflusst ebenfalls maßgeblich die Geschwindigkeit der Hefevermehrung und wird deshalb in die Ermittlung der Wachstumsrate innerhalb des Propagationsmodells einbezogen.

Die Lag-Phase betrifft schließlich eine Verzögerungsphase zu Beginn der Hefepropagation, welche insbesondere bei einer Überimpfung der Hefekultur in die Würze auftritt. Während dieser Phase sind die Hefezellen zwar biochemisch aktiv, jedoch teilen sie sich nicht. Somit ist auch die Einbeziehung der Dauer der Lag-Phase zur genauen Vorhersage eines Endzeitpunkts für die Hefevermehrung vorteilhaft.

Erfindungsgemäß wird anhand des Propagationsmodells ein Referenzwert für den Extraktgehalt ermittelt. Der Referenzwert kann beispielsweise kontinuierlich oder zu vorgebbaren Zeitpunkten während der Hefepropagation ermittelt werden.

Erfindungsgemäß wird der Istwert für den Extraktgehalt mit dem Referenzwert für den Extraktgehalt verglichen, wobei im Falle, dass eine Abweichung zwischen Istwert und Referenzwert einen vorgebbaren Grenzwert übersteigt, zumindest eine Einflussgröße für die Hefevermehrung in Abhängigkeit der Abweichung variiert wird. Idealerweise wird der Zeitpunkt der Ermittlung des Istwerts für die Ermittlung des Referenzwerts für den Extraktgehalt berücksichtigt bzw. beide Werte werden für den gleichen Zeitpunkt ermittelt. Der Vergleich ermöglicht also eine kontinuierliche Überwachung und/oder Steuerung des Prozesses.

In einer erfindungsgemäßen Variante wird während der Hefevermehrung eine Zufuhr von Sauerstoff geeignet eingestellt, insbesondere geregelt oder gesteuert werden. In einer anderen erfindungsgemäßen Variante wird der anhand des Propagationsmodells vorhergesagte Endzeitpunkt für die Hefevermehrung anhand der jeweiligen Gegebenheiten angepasst bzw. geändert.

So ist es beispielsweise denkbar, eine Temperatur des Gemischs während der Hefevermehrung anhand der Abweichung zwischen Istwert und Referenzwert des Extraktgehalts einzustellen, insbesondere so zu regeln oder zu steuern.

Ebenfalls beinhaltet eine Ausgestaltung, dass eine Belüftung des Gemischs während der Hefevermehrung, anhand der Abweichung zwischen Istwert und Referenzwert des Extraktgehalts und des Gehalts an gelöstem Sauerstoff eingestellt, insbesondere geregelt oder gesteuert, wird.

Eine Ausgestaltung des Verfahrens beinhaltet, dass eine Anstellkonzentration der Hefe zum Startzeitpunkt bestimmt wird.

In diesem Zusammenhang ist es von Vorteil, wenn anhand des Propagationsmodells, der Anstellkonzentration und eines Zielwerts für die Hefekonzentration, welche mit dem Zielwert für den Restextraktgehalt in Zusammenhang steht, eine Biomassenkonzentration der Hefe ermittelt wird.

Es ist in diesem Zusammenhang ebenfalls von Vorteil, wenn anhand der Biomassekonzentration eine viable Hefezellkonzentration während der Propagation ermittelt wird. Dies erlaubt vorteilhaft eine Überwachung der Zellkonzentration ohne eine direkte Messung.

Eine weitere Ausgestaltung beinhaltet schließlich, dass ein pH-Wert des Gemischs ermittelt wird. Dies wiederum dient der Überwachung der mikrobiologischen Sicherheit des Prozesses.

Die verschiedenen Einflussgrößen, welche bei der Hefevermehrung eine Rolle spielen, wie beispielsweise der Extraktgehalt, der FAN-Gehalt, die Temperatur, der Alkohol- bzw. Ethanolgehalt, der Gehalt an gelöstem Sauerstoff, oder der pH-Wert, können jeweils zu bestimmten vorgebbaren Zeitpunkten oder kontinuierlich bestimmt werden. Es ist sowohl eine Bestimmung einzelner der Einflussgrößen mittels einer Probennahme und nachfolgenden Analyse denkbar als auch die direkte Bestimmung im Prozess. Zur direkten Messung können geeignete Sensoren zur Bestimmung der jeweiligen Einflussgröße an oder in einem Behälter, welcher zur Durchführung der Hefevermehrung verwendet wird, angebracht werden. Dabei kann es sich um Thermometer, Sauerstoff- und/oder pH Sensoren handeln. Zur Bestimmung von Dichte und/oder Viskosität und mit von diesen Größen abhängigen Einflussgrößen, wie beispielsweise dem Alkoholgehalt, sei in diesem Zusammenhang beispielsweise auf die Vorrichtungen und Verfahren, welche in DE 10 2018 127 526 A1, DE 10 2016 120 326 A1, DE 10 2016 111 134 A1 oder DE 10 2015 112 421 A1 oder auch DE 10 2014 119 061 A1 Bezug genommen.

Anhand des Propagationsmodells ist es möglich, einen unter gegebenen Bedingungen idealen Verlauf für die Hefepropagation in Bezug auf die Zeitdauer und die gewünschte Zellzahl lebender Zellen hin vorzusagen und durch die parallele Bestimmung zumindest einer Einflussgröße geeignet zu steuern.

Das Verfahren kann sowohl auf einer Recheneinheit bzw. einem Computer am Ort der durchgeführten Hefevermehrung, also beispielsweise in der Brauerei durchgeführt werden. Es kann aber auch mittels einer externen Recheneinheit durchgeführt werden, sofern notwendige Messdaten an diese Recheneinheit übertragen werden. Beispielsweise kann das Verfahren durch eine Cloud-Anwendung implementiert werden. In diesem Falle sind die Messeinrichtungen zur Bestimmung von Werten der zuvor genannten Einflussgrößen, wie beispielsweise ein Thermometer oder ein Sauerstoffsensor, vorzugsweise mit Mitteln zur Übertragung von Messwerten an die Cloud ausgestaltet.

Die Erfindung sowie ihre vorteilhaften Ausgestaltungen werden anhand der nachfolgenden Figuren näher erläutert. Es zeigt:

Fig. 1: beispielhaft einen Behälter zur Durchführung einer Hefevermehrung und
Fig. 2: den Restextrakt und die Biomassekonzentration jeweils in Form von Messkurven und mittels des Propagationsmodells berechneten Referenzkurven.

In den Figuren werden gleiche Elemente mit demselben Bezugszeichen versehen.

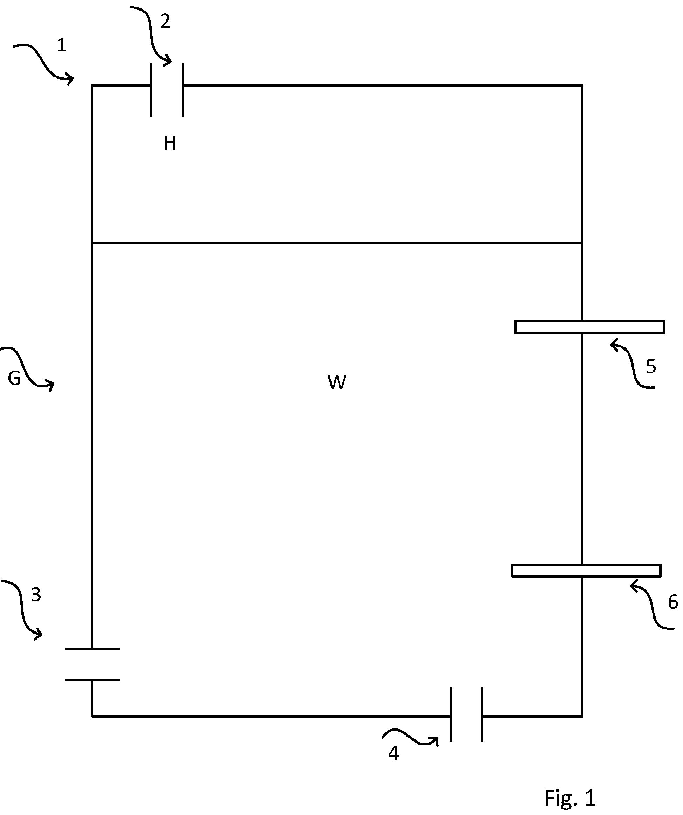

In Fig. 1 ist eine schematische und beispielhafte Abbildung eines Behälters 1 für eine Hefepropagation in einer Brauerei gezeigt. Der Behälter 1 umfasst einen Einlass 2 zum Ansetzen der gehopften Würze W mit Hefe H. Vorteilhaft wird zu Beginn der Hefevermehrung die Anstellkonzentration der Hefe H, die Temperatur T innerhalb des Behälters 1 und ggf. der FAN-Gehalt der Hefe H ermittelt. Nachfolgend beginnt für das Gemisch G der Prozess der Hefevermehrung.

Der Behälter 1 umfasst ferner einen Auslass 3 zur Entnahme und eine Einrichtung 4 zur Belüftung des Behälters 1. Zur Überwachung und/oder Steuerung sind für das hier gezeigte Beispiel ferner ein Sauerstoffsensor 5 und ein Thermometer 6 in den Tank eingebracht. Zur Einstellung der Temperatur T im Behälter 1 während der Hefevermehrung kann ferner eine Heiz-/Kühleinrichtung [nicht dargestellt] vorhanden sein.

Die vorliegende Erfindung stellt eine Möglichkeit zur Überwachung und/oder Steuerung der Hefevermehrung zur Verfügung. Es wird ein Wachstumsprozess unter kontrollierten Bedingungen und ein Ernten der Hefe zum richtigen Zeitpunkt, d.h. bei möglichst hoher Zellzahl und Vitalität der Hefe ermöglicht.

Aus dem Stand der Technik sind bereits zahlreiche Beschreibungen verschiedener Einflussgrößen der Hefevermehrung, sowie Modelle zur Beschreibung des Prozesses der Hefevermehrung bekannt, wie beispielsweise aus der Dissertation "Mathematically Based Management of Saccharomyces sp. Batch Propagation and Fermentations" von T. Kurz (2002) an der Technischen Universität München, oder in "Modeling of the Bacterial Growth Curve" von M.H. Zwietering et al. in Applied and Environmental Microbiology p.1875-1881, 1990. Da es sich bei der Hefevermehrung jedoch um ein komplexes, multivariantes Problem handelt, bedienen sich die verfügbaren Modelle an einer Vielzahl von Variablen bzw. unterschiedlichen Einflussgrößen und sind für eine praktische Steuerung und/oder Überwachung der Hefepropagation nur bedingt geeignet. Zudem sind viele der Einflussgrößen nur schwer direkt, insbesondere kontinuierlich, während der Hefevermehrung verfügbar. Die vorliegende Erfindung betrifft somit eine Vereinfachung der bekannten Modelle und Beschreibungen, welche eine zielgerichtete Steuerung und/oder Überwachung einer Hefevermehrung erlaubt. Eine wesentliche, der Erfindung zugrunde liegende Kenntnis betrifft eine zielgerichtete Auswahl von wesentlichen Einflussgrößen bei der Erstellung des Propagationsmodells.

Ausgangspunkt zur Modellierung der Hefevermehrung ist ein logistisches Propagationsmodell, wie in (Speers, et al., 2003) beschrieben, welches die Extraktabnahme S in Abhängigkeit der Zeit t, einer Substrataufnahmerate $\mu_s$, einer Extraktdifferenz $\Delta S = S_1 - S_2$ zwischen der Stammwürze $S_1$ und einem Zielwert für den Restextraktgehalt $S_2$ und einer Dauer für die Lag-Phase $\lambda$ beschreibt:

$$S(t) = S_2 - \frac{\Delta S}{1 + e^{(\mu_s(\lambda - t))}}$$

Die Bestimmung der Substrataufnahmerate $\mu_x$ basiert auf Anwendung der Monod-Kinetik zur Berücksichtigung einer Abhängigkeit der Wachstumsrate R von einer limitierenden Substratkonzentration während des Wachstumsprozesses durch eine Ausschöpfung der Nährstoffe, eine Ansammlung toxischer metabolische Produkte und durch den Ionenhaushalt. Wie in dem Artikel "Growth of Saccharomyces cerevisiae is controlled by its limited respiratory capacity: formulation and verification of a hypothesis" von B. Sonnenleitnert und O. Käppeli, erschienen in Biotechnology and Bioengineering, Vol. XXVIII, Pp. 927-937, John Wiley & Sons, Inc., 1986, beschrieben, ist die Substrataufnahmerate $\mu_x$ gegeben durch:

$$\mu_s = \mu_{s\,max} \cdot min\left(\frac{Z}{Z + K_Z}, \frac{N}{N + K_n}\right) \cdot \frac{K_E}{K_E + E}$$

Z beschreibt hier die Zuckerkonzentration, N die Stickstoffkonzentration, E den Ethanolgehalt, $\mu_{s,max}$ die maximale, spezifische Wachstumsrate und $K_s$ die halbmaximale Konzentration der Substrate.

Die Abhängigkeit von der Temperatur T beim Wachstum von Mikroorganismen als dominierender Einflussfaktor für den Wachstumsprozess erfolgt anhand einer in "Model for Bacterial Culture Growth Rate Throughout the Entire Biokinetic Temperature Range" von D.A. Ratowsky et al., erschienen in Journal of Bacteriology, p.1222-1226, 1983, beschriebenen erweiterten Form des Belehrädek-Modells für die Temperaturrate r:

$$\sqrt{r} = a * (T - T_{min}) * \left\{1 - e^{[b*(T - T_{max})]}\right\}.$$

Hier beschreiben $T_{min}$ bzw. $T_{max}$ eine minimale bzw. maximale Wachstumstemperatur und a, b empirische Parameter. Auf dieser Temperaturrate basierend, ergibt sich für die Dauer der Lag-Phase $\lambda$ in Abhängigkeit der Temperatur T:

$$\lambda = \left(a(T - T_{min}) \cdot \left\{1 - e^{[b \cdot (T - T_{max})]}\right\}\right)^{-2},$$

wie in "Modeling of Bacterial Growth with Shifts in Temperature" von M.H. Zwietering et al., Applied and Environmental Microbiology, p. 204-213, 1994, vorgeschlagen.

Damit kann der Extraktgehalt S als Funktion der Zeit t anhand des nachfolgenden Propagationsmodells S(t) ermittelt werden:

$$S(t) = S_2 - \frac{\Delta S}{1 + e^{-\left\langle \mu_{s\,max} \cdot min\left(\frac{S}{S+K_s}, \frac{N}{N+K_n}\right) \cdot \left(a \cdot (T - T_{min}) \cdot \left\{1 - e^{[b \cdot (T - T_{max})]}\right\}\right)^2 \right\rangle \left(t - \left(a \cdot (T - T_{min}) \cdot \left\{1 - e^{[b \cdot (T - T_{max})]}\right\}\right)^{-2} + c\right)}}$$

c stellt hierbei einen weiteren freien Parameter dar.

Anhand des erfindungsgemäßen Propagationsmodells S(t) ist es möglich, anhand der Stammwürze $E_1$, und der Temperatur T zu einem Startzeitpunkt $t_{start}$ einen theoretischen Endzeitpunkt $t_{end}$ für die Hefevermehrung zu ermitteln.

Die Extraktabnahme $\frac{dS(t)}{dt}$ steht mit dem Biomassezuwachs $\frac{dX(t)}{dt}$, also mit der Biomassekonzentration X(t) als Funktion der Zeit t in Zusammenhang. Die Extraktabnahme $\frac{dS(t)}{dt}$ entspricht der Nährstoffaufnahme der Hefe, weshalb die Biomassekonzentration X proportional mit der Abnahme des Extrakts S zunimmt. Der Proportionalitätsfaktor Y wird als Biomasseertrag bezeichnet und stellt die Menge an Biomasse pro Substrat dar. Die Rate des Biomassezuwachses $\frac{dX(t)}{dt}$ oder die Biomassewachstumsrate $\mu_x$ ergibt sich also anhand der Substrataufnahmerate $\mu_s$ zu:

$$\mu_X = Y \cdot \mu_S$$

Es ist also möglich, aus der Extraktdifferenz $\Delta E$ die Zunahme an Biomasse $\Delta X$ zu berechnen. Die Biomassekonzentration X bzw. die Hefekonzentration kann aus der Abnahme des Extrakts S ohne die Notwendigkeit, eine weitere direkte Messung vornehmen zu müssen, bestimmt werden. Auf diese Weise ist somit auch eine Zellkonzentrationsüberwachung möglich.

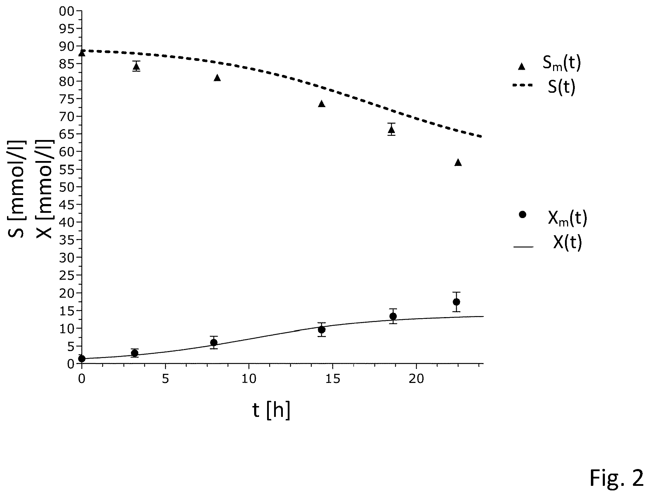

In Fig. 2 sind der Restextrakt S und die Biomassekonzentration X jeweils als Funktion der Zeit t in Form von in einer Versuchsmessung ermittelten Messwerten ($S_m(t)$ bzw. $X_m(t)$) und von mittels des Propagationsmodells S(t) bzw. X(t) berechneten Referenzkurven (gestrichelte und durchgezogene Linie) dargestellt. Für das das Propagationsmodell S(t) wurde in diesem Zusammenhang eine Extraktdifferenz $\Delta S=4\%$, während die gemessene Extraktdifferenz $\Delta S$ geringfügig größer war. Das Modell spiegelt in zufriedenstellender Art und Weise den Prozess der Hefevermehrung wider.

**Bezugszeichen**

1        Behälter

| 2 | Einlass |
|---|---|
| 3 | Auslass |
| 4 | Einrichtung zur Belüftung |
| 5 W | Sauerstoffsensor |
| 6 | Thermometer Würze |
| H | Hefe |
| T | Temperatur |
| FAN | FAN-Gehalt |
| G | Gemisch aus Würze und Hefe |
| S | Extraktabnahme |
| t | Zeit |
| $\Delta S$ | Extraktdifferenz |
| $S_1$ | Stammwürze |
| $S_2$ | Zielwert für den Restextraktgehalt |
| $\lambda$ | Dauer der Lag-Phase |
| $\mu_x$ | Substrataufnahmerate |
| R | Wachstumsrate |
| Z | Zuckerkonzentration |
| N | Stickstoffkonzentration |
| E | Ethanolgehalt |
| $\mu_{s,max}$ | Maximale spezifische Wachstumsrate |
| $K_s$ | Halbmaximale Konzentration der Substrate |
| $T_{min}$ | minimale Wachstumstemperatur |
| $T_{max}$ | maximale Wachstumstemperatur |
| a,b | empirische Parameter |
| S(t) | Propagationsmodell |
| $t_{start}$ | Startzeitpunkt |
| $t_{end}$ | Endzeitpunkt |
| X(t) | Biomassekonzentration |
| Y | Proportionalitätsfaktor, Biomasseertrag |
| $\mu_x$ | Biomassewachstumsrate |
| $\Delta X$ | Zunahme an Biomasse |
| $S_m(t)$ | Messwerte für den Extraktgehalt |
| $X_m(t)$ | Messwerte für die Biomassekonzentration |

## Patentansprüche

1. Verfahren, insbesondere computerimplementiertes Verfahren, zur Steuerung und/oder Überwachung einer Hefevermehrung, umfassend folgende Verfahrensschritte:

    - Bereitstellen eines Gemischs (G) aus Hefezellen (H) und Würze (W),
    - Bestimmen einer Stammwürze ($S_1$) der Würze (W) oder des Gemischs (G) und einer Temperatur (T) des Gemischs (G) zu einem Startzeitpunkt ($t_{start}$),
    - Berechnen eines theoretischen Endzeitpunkts ($t_{end}$) für die Hefevermehrung anhand eines logistischen Propagationsmodells (S(t)) für die Hefevermehrung, anhand der Stammwürze ($S_1$) und anhand der Temperatur (T) zum Startzeitpunkt ($t_{start}$),
    - Ermitteln eines Referenzwerts für einen Extraktgehalt (S) anhand des Propagationsmodells (S(t)),
    - Bestimmen eines Istwerts für den Extraktgehalt (S) des Gemisches (G) während der Hefevermehrung,
    - Vergleichen des Istwerts für den Extraktgehalt (S) mit dem Referenzwert für den Extraktgehalt, und
    - Variierung zumindest einer Einflussgröße für die Hefevermehrung in Abhängigkeit von einer Abweichung zwischen dem Istwert für den Extraktgehalt (S) und dem Referenzwert in dem Fall, dass die Abweichung einen vorgebbaren Grenzwert übersteigt,

        wobei der anhand des Propagationssignals berechnete Endzeitpunkt der Hefevermehrung ($t_{end}$) als Einflussgröße geändert wird,
        oder
        wobei für eine Konzentration an gelöstem Sauerstoff als Einflussgröße eine Belüftung des Gemischs (G) während der Hefevermehrung anhand der Abweichung zwischen Istwert (S) und Referenzwert des Extraktgehalts und anhand eines während der Hefevermehrung bestimmten Gehalts an gelöstem Sauerstoff des Gemischs (G) eingestellt wird.

**2.** Verfahren nach Anspruch 1,
wobei ein freier Aminostickstoffgehalt (FAN) der Würze (W) bestimmt wird, und für die Berechnung des theoretischen Endzeitpunkts ($t_{end}$) anhand des Propagationsmodells (S(t)) berücksichtigt wird.

**3.** Verfahren nach Anspruch 1 oder 2,
wobei die Temperatur (T) des Gemisches (G) während der Hefevermehrung bestimmt wird.

**4.** Verfahren nach einem der Ansprüche 1 bis 3,
wobei ein Alkoholgehalt des Gemisches (G) während der Hefevermehrung bestimmt wird.

**5.** Verfahren nach einem der Ansprüche 1 bis 4,
wobei als Propagationsmodell ein Modell für einen Extraktgehalt (S(t)) in dem Gemisch (G) verwendet wird, wobei der Extraktgehalt (S(t)) anhand der Stammwürze ($S_1$) zum Startzeitpunkt ($t_{start}$), eines Zielwerts für den Restextraktgehalt ($S_2$) und unter Berücksichtigung einer Substrataufnahmerate ($\mu_s$), einer Temperaturrate (r) und einer Dauer für eine Lag-Phase ($\lambda$) während der Hefevermehrung ermittelt wird.

**6.** Verfahren nach einem der Ansprüche 1 bis 5,

wobei eine Anstellkonzentration der Hefe zum Startzeitpunkt ($t_{start}$) bestimmt wird,
wobei anhand des Propagationsmodells (S(t)), der Anstellkonzentration und eines Zielwerts für die Hefekonzentration, welche mit dem Zielwert für den Restextraktgehalt ($S_2$) in Zusammenhang steht, eine Biomassenkonzentration (X(t)) der Hefe ermittelt wird, und
wobei anhand der Biomassekonzentration (X(t)) eine viable Hefezellkonzentration während der Propagation ermittelt wird.

**7.** Verfahren nach einem der Ansprüche 1 bis 6,
wobei ein pH-Wert des Gemischs ermittelt wird.

**Claims**

**1.** A method, in particular a computer-implemented method, for controlling and/or monitoring yeast propagation, comprising the following process steps:

- Providing a mixture (G) of yeast cells (H) and wort (W),
- determining an original wort ($S_1$) of the wort (W) or of the mixture (G) and a temperature (T) of the mixture (G) at a start time ($t_{start}$),
- calculating a theoretical end time ($t_{end}$) for yeast propagation based on a logical propagation model (S(t)) for yeast propagation, using the original wort ($S_1$) and using the temperature (T) at the start time ($t_{start}$),
- determining a reference value for an extract content (S) based on the propagation model (S(t)),
- determining an actual value for the extract content (S) of the mixture (G) during yeast propagation,
- comparing the actual value for the extract content (S) with the reference value for the extract content, and
- varying between at least one influencing variable for yeast propagation depending on a deviation between the actual value for the extract content (S) and the reference value in the event that the deviation exceeds a specifiable limit value,

wherein the end time ($t_{end}$) for yeast propagation calculated based on the propagation signal is changed as an influencing variable,
or
wherein aeration of the mixture (G) during yeast propagation is set for a concentration of dissolved oxygen as influencing variable based on the deviation between the actual value (S) and reference value of the extract content and based on a content of dissolved oxygen in the mixture (G) determined during yeast propagation.

**2.** The method as claimed in claim 1,
wherein the free amino nitrogen (FAN) of the wort (W) is determined, and is taken into account for calculating the theoretical end time ($t_{end}$) using the propagation model (S(t)).

3. The method as claimed in claim 1 or 2,
   wherein the temperature (T) of the mixture (G) is determined during yeast propagation.

4. The method as claimed in one of claims 1 to 3,
   wherein an alcohol content of the mixture (G) is determined during yeast propagation.

5. The method as claimed in one of claims 1 to 4,
   wherein a model for an extract content (S(t)) in the mixture (G) is used as the propagation model, wherein the extract content (S(t)) is determined using the original wort ($S_1$) at the start time ($t_{start}$), a target value for the residual yeast content ($S_2$) and considering a substrate absorption rate ($\mu_s$), a temperature rate (r) and a duration of a lag phase ($\lambda$) during yeast propagation.

6. The method as claimed in one of claims 1 to 5,

   wherein a starting concentration of the yeast is determined at the start time ($t_{start}$), wherein a biomass concentration (X(t)) of the yeast is determined based on the propagation model (S(t)), the starting concentration and a target value for the yeast concentration, which is linked to the target value for the residual extract content ($S_2$), and
   wherein a viable yeast cell concentration is determined during propagation based on the biomass concentration (X(t)).

7. The method as claimed in one of claims 1 to 6, wherein a pH value of the mixture is determined.


**Revendications**

1. Procédé, notamment un procédé mis en œuvre par ordinateur, lequel procédé est destiné à la commande et/ou à la surveillance d'une multiplication de levure, lequel procédé comprend les étapes suivantes :

   - Préparation d'un mélange (G) de cellules de levure (H) et de moût (W),
   - Détermination d'un moût primitif ($S_1$) du moût (W) ou du mélange (G) et d'une température (T) du mélange (G) à un instant initial ($t_{start}$),
   - Calcul d'un instant final théorique ($t_{end}$) pour la multiplication de levure à l'aide d'un modèle de propagation logistique (S(t)) pour la multiplication de levure, à l'aide du moût primitif ($S_1$) et à l'aide de la température (T) à l'instant initial ($t_{start}$),
   - Détermination d'une valeur de référence pour une teneur en extrait (S) à l'aide du modèle de propagation (S(t)),
   - Détermination d'une valeur réelle pour la teneur en extrait (S) du mélange (G) pendant la multiplication de levure,
   - Comparaison de la valeur réelle pour la teneur en extrait (S) avec la valeur de référence pour la teneur en extrait, et
   - Variation d'au moins une grandeur d'influence pour la multiplication de levure en fonction d'un écart entre la valeur réelle pour la teneur en extrait (S) et la valeur de référence dans le cas où l'écart dépasse une valeur limite pouvant être prédéfinie,

   l'instant final de la multiplication de levure ($t_{end}$) calculé à l'aide du signal de propagation étant modifié en tant que grandeur d'influence,
   ou
   une aération du mélange (G) étant réglée - pour une concentration en oxygène dissous comme grandeur d'influence - pendant la multiplication des levures à l'aide de l'écart entre la valeur réelle (S) et la valeur de référence de la teneur en extrait et à l'aide d'une teneur en oxygène dissous du mélange (G) déterminée pendant la multiplication des levures.

2. Procédé selon la revendication 1,
   pour lequel une teneur en azote aminé libre (FAN) du moût (W) est déterminée, et est prise en compte pour le calcul de l'instant final théorique ($t_{end}$) à l'aide du modèle de propagation (S(t)).

3. Procédé selon la revendication 1 ou 2,

pour lequel la température (T) du mélange (G) est déterminée pendant la multiplication de la levure.

4. Procédé selon l'une des revendications 1 à 3,
pour lequel on détermine un taux d'alcool du mélange (G) pendant la multiplication de la levure.

5. Procédé selon l'une des revendications 1 à 4,
pour lequel on utilise comme modèle de propagation un modèle pour une teneur en extrait $(S(t))$ dans le mélange (G), la teneur en extrait $(S(t))$ étant déterminée à l'aide du moût primitif $(S_1)$ à l'instant initial $(t_{start})$, d'une valeur cible pour la teneur en extrait résiduel $(S_2)$ et en tenant compte d'un taux d'absorption de substrat $(\mu_s)$, d'un taux de température (r) et d'une durée pour une phase de latence $(\lambda)$ pendant la multiplication de levure.

6. Procédé selon l'une des revendications 1 à 5,

pour lequel on détermine une concentration d'ensemencement de la levure à l'instant initial $(t_{start})$,
pour lequel on détermine une concentration de biomasse $(X(t))$ de la levure à l'aide du modèle de propagation $(S(t))$, de la concentration d'ensemencement et d'une valeur cible pour la concentration de levure, laquelle est liée à la valeur cible pour la teneur en extrait résiduel $(S_2)$, et
pour lequel on détermine une concentration de cellules de levure viable pendant la propagation à l'aide de la concentration de biomasse $(X(t))$.

7. Procédé selon l'une des revendication 1 à 6,
pour lequel on détermine une valeur de pH du mélange.

Fig. 1

Fig. 2

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 102019110821 A1
- EP 2846160 A1
- US 20080109100 A1
- DE 102018127526 A1
- DE 102016120326 A1
- DE 102016111134 A1
- DE 102015112421 A1
- DE 102014119061 A1


**In der Beschreibung aufgeführte Nicht-Patentliteratur**

- **VON T. KURZ**. Mathematically Based Management of Saccharomyces sp. Batch Propagation and Fermentations. *Technischen Universität München*, 2002
- **VON M.H. ZWIETERING et al.** Modeling of the Bacterial Growth Curve. *Applied and Environmental Microbiology*, 1990, 1875-1881
- Growth of Saccharomyces cerevisiae is controlled by its limited respiratory capacity: formulation and verification of a hypothesis. **VON B. SONNEN-LEITNERT** ; **O. KÄPPELI**. Biotechnology and Bioengineering. John Wiley & Sons, Inc., 1986, vol. XXVIII, 927-937
- **VON D.A. RATOWSKY et al.** Model for Bacterial Culture Growth Rate Throughout the Entire Biokinetic Temperature Range. *Journal of Bacteriology*, 1983, 1222-1226
- **VON M.H. ZWIETERING et al.** Modeling of Bacterial Growth with Shifts in Temperature. *Applied and Environmental Microbiology*, 1994, 204-213